**Europäisches Patentamt**

**European Patent Office**

**Office européen des brevets**

(11) Veröffentlichungsnummer: **0 254 838**
**B1**

(12) **EUROPÄISCHE PATENTSCHRIFT**

(45) Veröffentlichungstag der Patentschrift:
**22.08.90**

(21) Anmeldenummer: **87107934.9**

(22) Anmeldetag: **02.06.87**

(51) Int. Cl.⁵: **A61G 12/00**, A61G 10/02,
A61G 7/05, A61G 11/00

(54) **Bestrahlungseinrichtung.**

(30) Priorität: **02.07.86 DE 3622148**

(43) Veröffentlichungstag der Anmeldung:
**03.02.88 Patentblatt 88/5**

(45) Bekanntmachung des Hinweises auf die Patenterteilung:
**22.08.90 Patentblatt 90/34**

(84) Benannte Vertragsstaaten:
**CH DE FR GB LI**

(56) Entgegenhaltungen:
**DE-A- 2 610 226**
**DE-U- 7 602 145**
**US-A- 1 914 288**
**US-A- 2 292 120**

(73) Patentinhaber: **Drägerwerk Aktiengesellschaft,
Moislinger Allee 53-55, D-2400 Lübeck 1(DE)**

(72) Erfinder: **Koch, Jochim, Dr., Alte Meierei,
D-2411 Hollenbek(DE)**
Erfinder: **Franz, Wolfgang, Ziegelstrasse 191,
D-2400 Lübeck(DE)**

ACTORUM AG

**Beschreibung**

Die Erfindung betrifft eine Bestrahlungseinrichtung zur Wärmeversorgung einer der Aufnahme von Personen dienenden Liegefläche, über welcher in räumlichem Abstand eine Wärmequelle angeordnet ist.

Derartige Bestrahlungseinrichtungen werden zur Warmhaltung von Frühgeborenen, Säuglingen oder Kleinkindern benutzt, welche auf einer von mehreren Seiten zugänglichen Liegefläche liegen und somit durch entsprechendes Personal ohne Behinderung gepflegt werden können.

Wegen der notwendigen Einsehbarkeit zum Patienten und um während der Behandlungsvorgänge am Patienten eine möglichst große Bewegungsfreiheit zu haben, werden diese Strahlungsheizungen nur in einem Teilbereich oberhalb der Liegefläche untergebracht.

So ist zum Beispiel in der DE-OS 29 13 282 ein Behandlungstisch für Neugeborene und Kleinkinder beschrieben, über dessen Mittenbereich eine längserstreckte, schmale Strahlungsheizung angeordnet ist. Eine solche bekannte Strahlungsheizung erwärmt den zentralen Teilbereich der Liegefläche intensiv, während infolge der radial abnehmenden Wärmeenergie zum Randbereich der Liegefläche hin dort zu wenig Strahlungsenergie auftrifft, um den Patienten mit ausreichender Wärmeenergie zu versorgen. Eine gleichmäßige Bestrahlung der gesamten Liegefläche mit auf ihr gleich verteilter Wärmeenergie ist aber deswegen erforderlich, weil die zu pflegende Person aufgrund ihrer Größe unterschiedliche Stellen der Liegefläche bedeckt und weil sie während der Behandlung an unterschiedlichen Stellen der Liegefläche gelagert werden muß.

Eine weitere bekannte Strahlungsheizung (DE-GM 76 01 145) ist über der Liegefläche derart angeordnet, daß der Wärmestrahler flächig ausgebildet ist und in seinen Abmessungen mit den Dimensionen der Liegefläche nahezu übereinstimmt. Diese bekannte Lösung weist jedoch den Nachteil auf, daß das Pflegepersonal, welches von den freien Seitenflächen her Zugang zu der auf der Liegefläche befindlichen Person haben muß, sich während seiner Behandlungstätigkeit über die Liegefläche beugen muß, wodurch der Kopfbereich des Personals unmittelbar der Strahlungswärme ausgesetzt ist. Dies führt bei länger andauernder Behandlung zu unerträglicher Wärmebelästigung des Pflegepersonals und schattet den Patienten ab.

Die vorliegende Erfindung geht somit von der Aufgabenstellung aus, eine Bestrahlungseinrichtung der genannten Art so zu verbessern, daß sie eine gleichmäßige Bestrahlung der Liegefläche ermöglicht, bei der die Zugangsseiten von dem Strahlungsweg freigehalten sind.

Die Lösung der Aufgabe erfolgt dadurch, daß kleinflächige Wärmestrahler vorgesehen sind, welche in vertikalem räumlichem Abstand über den Eckpunkten eines den Liegebereich umfassenden Vielecks angebracht sind.

Der Vorteil der Erfindung ist im wesentlichen darin zu sehen, daß unabhängig von der größeren geometrischen Form der Liegefläche die Wärmestrahler über den Eckpunkten des Vielecks angeordnet sind und jeder von dort aus die Liegefläche mit einem Strahlungskegel bestrahlt, welcher genügend Freiraum für den Zugang des Bedienungspersonals läßt.

Ein weiterer Vorteil der Erfindung liegt darin, daß die Bestrahlung der Person auf der Liegefläche von den Eckpunkten aus anteilig mehr auf die Seitenflächen des Körpers der zu bestrahlenden Person ausrichtbar ist, als es bei den bekannten Bestrahlungseinrichtungen der Fall ist. Dazu braucht lediglich der Abstand der Wärmestrahler von dem Liegebereich entsprechend verändert zu werden. Je nach Lageort der Person können einzelne Strahler durch geringe Schwenkbewegungen gezielt auf sie gerichtet werden.

Zur weiteren Unterstützung der Wärmebestrahlung kann vorteilhafterweise ein zusätzlicher Wärmestrahler im Mittenbereich über dem Vieleck vorgesehen sein.

Zweckmäßigerweise kann der Platz über dem Mittenbereich des Vielecks zur Aufnahme einer zusätzlichen Bestrahlungslampe vorgesehen sein, deren Strahlungsintensität in einem anderen Wellenlängenbereich liegt als demjenigen der übrigen Wärmestrahler. Dies kann zum Beispiel ein Blaustrahler sein, welcher zur Durchführung einer Fototherapie bei Früh- und Neugeborenen geeignet ist. Dadurch ist eine Wärmestrahlung möglich mit der Kombination einer Fototherapie mit dem Vorteil der großflächigen Bestrahlung des Patienten.

Vorteilhafterweise können die Wärmestrahler mit Lichtstrahlern für weißes Licht kombiniert sein. Dadurch ist eine naturgetreue Beobachtung und Ausleuchtung der Liegefläche und des Patienten möglich.

Es hat sich als besonders zweckmäßig erwiesen, als Wärmestrahler Keramik-Hochtemperaturstrahler einzusetzen, die eine geringe, hochtemperierte Strahlungsfläche aufweisen, welche je nach Bedarf unterschiedlich in ihrer Abstrahlrichtung ausrichtbar sind.

Ein Ausführungsbeispiel der Erfindung wird anhand der schematischen Zeichnung dargestellt und im folgenden näher erläutert:

Es zeigen:

Fig. 1 die Draufsicht auf eine Liegefläche

Fig. 2 die Ansicht der Liegefläche mit Wärmestrahlern.

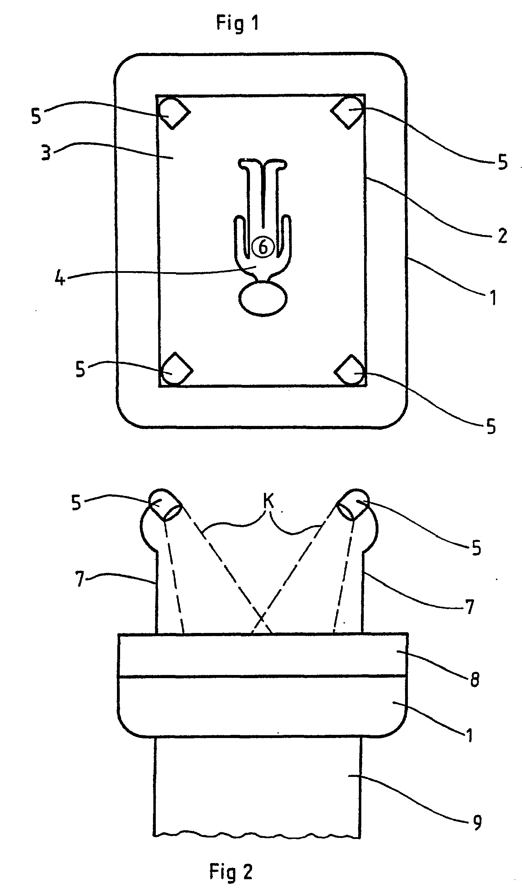

In Fig. 1 ist die Draufsicht auf eine Liegefläche (1) gezeigt, auf deren Mitte die Konturen einer Person (4) schematisch gezogen sind. Der Liegebereich (3) der Person (4) ist von einem als Viereck dargestellten Vieleck umrandet. In den Ecken des Vielecks (2) sind Wärmestrahler (5) angebracht, deren Strahlungsrichtung auf den Körper der Person (4) ausgerichtet sind. Über den Mittenbereich des Vielecks (2) ist ein weiterer Strahler (6) als Wärmestrahler angeordnet, welcher den Mittenbereich der Liegefläche (1) direkt bestrahlt.

In Fig. 2 ist die Teilansicht der Liegefläche (1) von einer ihrer Stirnseiten aus gesehen dargestellt. Die Liegefläche (1) ist mit einer Umrandung (8) umgeben und ruht auf einem Fuß (9). Über der Liegefläche (1) sind über Ständer (7) die Wärmestrahler (5) gehalten, welche in ihrer Strahlungsrichtung (K) auf den Liegebereich (3) ausgerichtet sind.

Durch die Anordnung der Wärmestrahler (5) in den Ecken des Vielecks (2) bleiben dessen Seitenlinien frei von Wärmestrahlung, so daß sich in Höhe der Wärmestrahler (5) eine strahlungsfreie Zone ausbildet, in welcher das Bedienungspersonal hinzutreten kann, ohne daß es im Kopfbereich zu einer belästigenden Wärmebestrahlung kommt.

**Patentansprüche**

1. Bestrahlungseinrichtung zur Wärmeversorgung einer der Aufnahme von Personen dienenden Liegefläche, über welcher in räumlichem Abstand eine Wärmequelle angeordnet ist, dadurch gekennzeichnet, daß kleinflächige Wärmestrahler (5) vorgesehen sind, welche in vertikalem räumlichem Abstand über den Eckpunkten eines den Liegebereich (3) umfassenden Vielecks (2) angebracht sind.

2. Bestrahlungseinrichtung nach Anspruch 1, dadurch gekennzeichnet, daß mindestens ein zusätzlicher Wärmestrahler (6) über dem Mittenbereich des Vielecks (2) angebracht ist.

3. Bestrahlungseinrichtung nach Anspruch 1, dadurch gekennzeichnet, daß über dem Mittenbereich des Vielecks (2) ein Blaustrahler (6) angebracht ist, dessen maximale Strahlungsintensität im Wellenlängenbereich von 400 bis 550 nm liegt.

4. Bestrahlungseinrichtung nach einem der Ansprüche 1 und 2, dadurch gekennzeichnet, daß die Wärmestrahler (5,6) mit Lichtstrahlern kombiniert sind.

**Claims**

1. A radiation device for supplying heat to a reclining surface used for receiving people, above which reclining surface a heat source is arranged at a distance, characterised in that heat radiators (5) are provided which have a small surface area and which are arranged at a vertical distance above the corner points of a polygon (2) surrounding the reclining area (3).

2. A radiation device according to claim 1, characterised in that at least one additional heat radiator (6) is arranged above the central region of the polygon (2).

3. A radiation device according to claim 1, characterised in that a blue radiator (6), whose maximum radiation intensity lies in the wavelength band of 400 to 550 nm, is arranged above the central region of the polygon (2).

4. A radiation device according to one of claims 1 and 2, characterised in that the heat radiators (5, 6) are combined with light radiators.

**Revendications**

1. Dispositif d'irradiation pour l'alimentation en chaleur d'une surface de repos servant à recevoir des personnes, au-dessus de laquelle une source de chaleur est disposée, à une certaine distance, caractérisé en ce qu'on prévoit des radiateurs thermiques (5) de petite surface qui sont placés, à une certaine distance verticale, au-dessus des angles d'un polygone (2) entourant la zone de repos (3).

2. Dispositif d'irradiation selon la revendication 1, caractérisé en ce qu'on place au moins un radiateur thermique (6) supplémentaire au-dessus de la zone centrale du polygone (2).

3. Dispositif d'irradiation selon la revendication 1, caractérisé en ce qu'on place, au-dessus de la zone centrale du polygone (2), un émetteur de lumière bleue (6) dont l'intensité maximale de rayonnement se situe dans la gamme de longueurs d'onde comprises entre 400 et 500 nm.

4. Dispositif d'irradiation selon la revendication 1 ou 2, caractérisé en ce que les radiateurs thermiques (5, 6) sont combinés à des émetteurs de rayonnement lumineux.

EP 0 254 838 B1

Fig 1

5
3
5
4
6
2
1
5
5

5
K
5
7
7
8
1
9

Fig 2